# EUROPEAN PATENT SPECIFICATION

(11) **EP 2 679 166 B1**
(45) Date of publication and mention of the grant of the patent: **30.03.2016**
(21) Application number: 13163308.3
(22) Date of filing: 11.04.2013
(51) Int. Cl.: A61B 8/08, A61B 8/00, A61B 6/00, A61B 5/00, A61B 5/055, G06T 7/00

(54) **Diagnosis image apparatus and operation method thereof**
Diagnosebildvorrichtung und Betriebsverfahren dafür
Appareil d'image de diagnostic et son procédé de fonctionnement

(30) Priority: 28.06.2012 KR 20120070231
(43) Date of publication of application: 01.01.2014
(73) Proprietor: Samsung Medison Co., Ltd., Gangwon-do 250-870 (KR)
(72) Inventor: Lee, Bong-heon, 250-870 Gangwon-do (KR); Lee, Jin-yong, 250-870 Gangwon-do (KR); Park, Sung-wook, 250-870 Gangwon-do (KR)
(74) Representative: Schmid, Wolfgang

(56) References cited:
- WO-A2-01/16886
- WO-A2-99/49775
- JP-A- 2005 218 713
- US-A1- 2006 058 610
- US-A1- 2006 170 714

## Description

### BACKGROUND OF THE INVENTION

### 1. Field of the Invention

The present invention relates to a diagnosis imaging apparatus and an operation method thereof.

### 2. Description of the Related Art

Diagnosis imaging apparatuses refer to medical imaging apparatuses such as ultrasound imaging apparatuses, computed tomography (CT) apparatuses, or magnetic resonance imaging (MRI) apparatuses.

A diagnosis imaging apparatus may acquire a first image of the heart of a subject when the subject is subjected to low intensity stress, and acquire a second image of the heart when the subject is subjected to high intensity stress. A user may diagnose if the subject has an illness in the heart by comparing the first image with the second image. This is because changes in cardiac muscle thickness when the subject is subjected to stress at different intensity levels depend on whether the subject has an illness in the heart or not.

However, when the user diagnoses an illness by separately viewing the first and second images, the user may not readily perceive a difference between the first and second images during the diagnosis, which may reduce diagnosis accuracy. To increase the diagnosis accuracy, the user may need to perform an additional task, such as reconfirming the first and second images. However, the additional task may delay the diagnosis time.

Therefore, there is a demand for an efficient diagnosis imaging apparatus and an operation method thereof.

JP 2005 218713 A refers to an ultrasonic diagnostic system in which a displacement history image and a histogram when the load is not applied are arranged to the left side of a screen. Furthermore, a displacement history image and a histogram when the load is applied are also arranged to the right side of the screen. A dashed line for indicating the division areas of area calculation is displayed by superimposition in the respective displacement history images. In the histograms the numbers of the division areas are arranged in a horizontal axis, and areas corresponding to each division area are indicated in a vertical axis.

### SUMMARY OF THE INVENTION

The present invention provides an efficient diagnosis imaging apparatus according to claim 1 and an operation method thereof according to claim 10.

According to an aspect of the present invention, there is provided a diagnosis imaging apparatus including: an image processing apparatus for acquiring a first image including a first contour of a heart wall based on first image data of a heart of a subject acquired when the subject is subjected to a first stress intensity level, acquiring a second image including a second contour of the heart wall based on second image data of the heart of the subject acquired when the subject is subjected to a second stress intensity level, and acquiring a third image representing a difference between the first contour and the second contour based on the first image and the second image; and a display apparatus for displaying the third image.

According to another aspect of the present invention, there is provided an operation method of a diagnosis imaging apparatus, the method including: acquiring a first image including a first contour of a heart wall based on first image data of a heart of a subject acquired when the subject is subjected to a first stress intensity level; acquiring a second image including a second contour of the heart wall based on second image data of the heart of the subject acquired when the subject is subjected to a second stress intensity level; acquiring a third image representing a difference between the first contour and the second contour based on the first image and the second image; and displaying the third image.

According to another aspect of the present invention, there is provided a display apparatus displaying a third image representing a difference between a first contour and a second contour of a heart wall, the first contour of the heart wall being based on first image data of a heart of a subject acquired when the subject is subjected to a first stress intensity level, and the second contour being based on second image data of the heart of the subject acquired when the subject is subjected to a second stress intensity level.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of a diagnosis imaging apparatus according to an embodiment of the present invention;
FIG. 2 illustrates a first image and a second image acquired by an image processing apparatus of FIG. 1, according to an embodiment of the present invention;
FIG. 3 illustrates a third image displayed on a display apparatus of FIG. 1, according to an embodiment of the present invention;
FIG. 4 illustrates a third image displayed on the display apparatus of FIG. 1, according to another embodiment of the present invention;
FIG. 5 is a view illustrating changes in thickness of a cardiac muscle according to types of heart conditions and stress levels;
FIG. 6 illustrates first to third images displayed on the display apparatus of FIG. 1, according to another embodiment of the present invention;
FIG. 7 illustrates first to third images displayed on the display apparatus of FIG. 1, according to another embodiment of the present invention;
FIG. 8 illustrates first to fourth images displayed on the display apparatus of FIG. 1, according to another embodiment of the present invention;
FIG. 9 illustrates first to third images displayed on the display apparatus of FIG. 1, according to another embodiment of the present invention; and
FIG. 10 is a flowchart of an operation method of a diagnosis imaging apparatus, according to an embodiment of the present invention.

### DETAILED DESCRIPTION OF THE INVENTION

As used herein, the term "and/or" includes any and all combinations of one or more of the associated listed items. Expressions such as "at least one of," when preceding a list of elements, modify the entire list of elements and do not modify the individual elements of the list.

Hereinafter, embodiments of the present invention are described in detail with reference to the appended drawings.

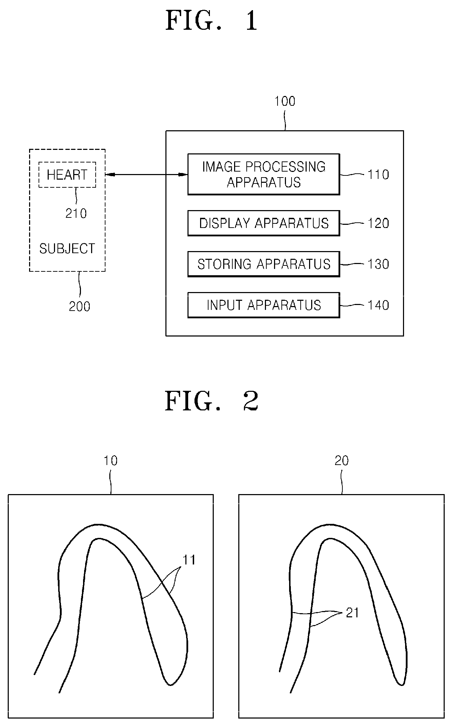

FIG. 1 is a block diagram of a diagnosis imaging apparatus 100 according to an embodiment of the present invention.

Referring to FIG. 1, the diagnosis imaging apparatus 100 includes an image processing apparatus 110 and a display apparatus 120. The diagnosis imaging apparatus 100 may further include a storing apparatus 130 and an input apparatus 140. A subject 200 may be an animal body, including a human body, having a heart 210.

The diagnosis imaging apparatus 100 is an apparatus for diagnosing whether the subject 200 has an illness in the heart 210, based on image data of the heart 210 of the subject 200. In some embodiments, the diagnosis imaging apparatus 100 may be a medical imaging apparatus, such as an ultrasound imaging apparatus, a computed tomography (CT) apparatus, or magnetic resonance imaging (MRI) apparatus.

The image processing apparatus 110 may acquire first image data of the heart 210 of the subject 200 when the subject 200 is subjected to a first stress intensity level, and may acquire second image data of the heart 210 when the subject 200 is subjected to a second stress intensity level higher than the first intensity level. The first image data and the second image data are obtained by subjecting the subject 200 to different stress intensity levels. The first image data and second image data may be 2-dimensional (2D) image data or 3-dimensional (3D) volume data, but are not limited thereto. The storing apparatus 130 may store the first image data and the second image data.

The intensity level of stress may be controlled by adjusting the intensity of exercise the subject 200 does or a dose of drug administered to the subject 200. For example, the first image data may be image data acquired from the subject 200 to which a first drug dosage is administered in a relaxed state, and the second image data may be image data acquired from the subject 200 to which a second drug dosage higher than the first drug dosage is administered in a relaxed state. The drug administered to the subject 100 may be dobutamine. In some other embodiments, the first image data may be image data acquired while the subject 200 is exercising at a first exercise intensity level, and the second image data may be image data acquired while the subject 200 is exercising at a second intensity level higher than the first exercise intensity level. For example, the exercise at the first intensity level may be riding a bike, and the exercise at the second intensity level may be walking or running.

The image processing apparatus 110 may acquire a first image including a first contour of the heart wall of the heart 210 based on the first image data, and acquire a second image including a second contour of the heart wall of the heart 210 based on the second image data.

FIG. 2 illustrates a first image 10 and a second image 20 acquired by the image processing apparatus 110 of FIG. 1, according to an embodiment of the present invention.

Referring to FIGS. 1 and 2, the first image 10 may include a first contour 11 of the heart wall of the heart 210 when the subject 200 is subjected to a first stress intensity level. The second image 21 may include a second contour 21 of the heart wall of the heart 210 when the subject 200 is subjected to a second stress intensity level higher than the first stress intensity level.

The first image 10 may be an image including the first contour 11 of the heart wall based on a cross-sectional image of the heart 210 acquired from the first image data, and the second image 20 may be an image including the second contour 21 of the heart wall based on a cross-sectional image of the heart 210 acquired from the second image data. The cross-sectional image of the heart 210 may be a 4-chamber view, a 3-chamber view, or a 2-chamber view. Each of the first contour 11 and the second contour 21 may represent the heart wall of the left ventricle.

Although in FIG. 2 the first image 10 and the second image 20 are illustrated as only including the first contour 11 and the second contour 21, respectively, the embodiment is not limited thereto. In some other embodiments, the first image 10 and the second image 20 may be images with the first contour 11 and the second contour 21 on cross-sections of the heart, respectively.

The first contour 11 and the second contour 21 may be automatically or manually drawn on the first image 10 and the second image 20, respectively. For example, the first contour 11 and the second contour 21 may be automatically drawn based on brightness. In some other embodiments, the first contour 11 and the second contour 21 may be drawn based on a user input via the input apparatus 140. The input apparatus 140 may be, for example, a touch panel on the display apparatus 120. A user may draw contours of the heart wall on a displayed cross-sectional image of the heart by using the input apparatus 140, so that the first contour 11 and the second contour 21 may be included in the first image and the second image 20, respectively.

The image processing apparatus 110 may acquire a third image representing a difference between the first contour 11 and the second contour 21 based on the first image 10 and the second image 20. The display apparatus 120 may display the third image.

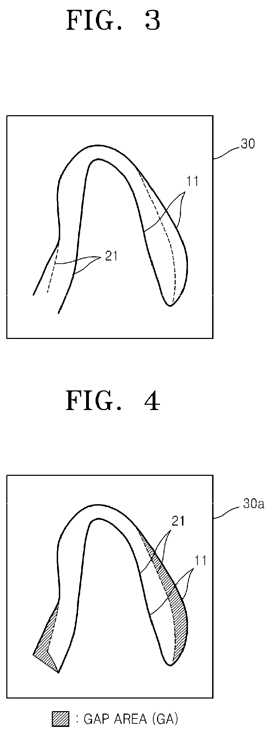

FIG. 3 illustrates a third image 30 displayed on the display apparatus 120 of FIG. 1, according to another embodiment of the present invention.

Referring to FIGS. 2 and 3, the first image 10 including the first contour 11 and the second image 20 including the second contour 21 are overlapped on the third image 30 so that a difference between the first contour 11 and the second contour 21 is represented on the third image 30. The first contour 11 and the second contour 12 in the third image 30 may be represented by, for example, a solid line and a dashed line, respectively, to be distinguished from each other.

Unlike in FIG. 3, the first contour 11 and the second contour 21 may be represented in different colors to be distinguished from each other. The third image 30 may represent the difference between the first contour 11 and the second contour 21 in any of a variety of ways, and thus, the third image 30 is not limited to the above.

Therefore, the user may easily perceive the difference between the first contour 11 and the second contour 21 on the first image 30.

FIG. 4 illustrates a third image 30a displayed on the display apparatus 120 of FIG. 1, according to another embodiment of the present invention.

Referring to FIGS. 2 and 4, the first image 10 including the first contour 11 and the second image 20 including the second contour 21 are overlapped on the third image 30 so that a difference between the first contour 11 and the second contour 21 is represented on the third image 30a. A non-overlapping heart wall area between the first contour 11 and the second contour 21 in the third image 30a, i.e., a gap area GA, may be displayed to be distinguished from other areas. In some embodiments, the gap area GA in the third image 30a may be represented hatched or in a different color to be distinguished from the other areas. Therefore, the user may easily perceive the difference between the first contour 11 and the second contour 21 on the third image 30a.

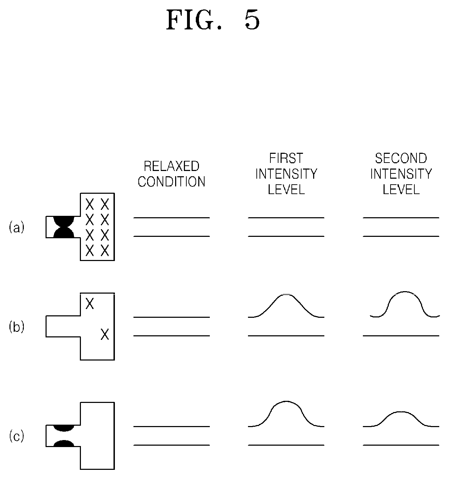

FIG. 5 is a view illustrating changes in thickness of a cardiac muscle according to types of heart conditions and stress levels. In particular, (A) to (C) in FIG. 5 illustrate different types of heart conditions of a subject observed to have changes in thickness of the cardiac muscle when the subject is in a relaxed state, is subjected to a first stress intensity level, or is subjected to a second intensity stress level.
(A) in FIG. 5 illustrates a heart condition with involving contraction of the coronary arteries of the subject and severe cardiac muscle damage. In this case, no change in thickness of the cardiac muscle was found in a relaxed state or when the subject is subjected to the first stress intensity level or the second stress intensity level.
(B) in FIG. 5 illustrates a heart condition with no contraction of the heart coronary arteries of the subject and nearly zero cardiac muscle damage. The thickness of the cardiac muscle increased when the subject is subjected to the first intensity stress level or the second stress intensity level higher than the first intensity stress level, as compared with the thickness of the cardiac muscle in a relaxed state.
(C) in FIG. 5 illustrates a heart condition with contraction of the coronary arteries of the subject and no cardiac muscle damage. The thickness of the cardiac muscle increased when the subject is subjected to the first stress intensity level or the second stress intensity level higher than the first stress intensity level, as compared with the thickness of the cardiac muscle in a relaxed state, with a greater increase in thickness of the cardiac muscle when the first stress intensity level lower than the second stress intensity level is applied, as compared with when the second stress intensity level is applied.

Therefore, whether the heart has an illness, such as cardiac muscle damage, contraction of the heart coronary arteries, or like, may be diagnosed by comparing changes in thickness of the cardiac muscle after subjecting the subject to stress at different intensity levels.

Referring back to FIGS. 1 to 3, the user may intuitively perceive the difference in heart wall thickness of the subject on the third image 30 that represents the difference between the first contour 11 and the second contour 21 displayed on the display apparatus 120. Therefore, the user may be convenienced in diagnosing the heart 210 of the subject 200, and a diagnosis speed may be improved. Since the third image 30 represents the difference between the first contour 11 and the second contour 21, this may ensure an objective comparison between the thicknesses of the heart wall when the subject 200 is subjected to stress at different intensity levels, and diagnosis accuracy may be increased.

If only the first image 10 and/or the second image 20, excluding the third image 30, are displayed on the display apparatus 120, the user may not be able to readily perceive a difference in heart wall thickness when the subject 200 is subjected to stress at different intensity levels. Thus, diagnosis accuracy may be reduced. To increase diagnosis accuracy, an additional task that, for example, the user reconfirms the first image 10 and/or the second image 20 may be performed. However, such an additional task may delay the diagnosis time.

Therefore, according to the above embodiments of the present invention, the diagnosis imaging apparatus 100 may provide user convenience to diagnose and may improve the diagnosis speed.

The display apparatus 120 may also display the first image 10 and the second image 20 along with the third image 30.

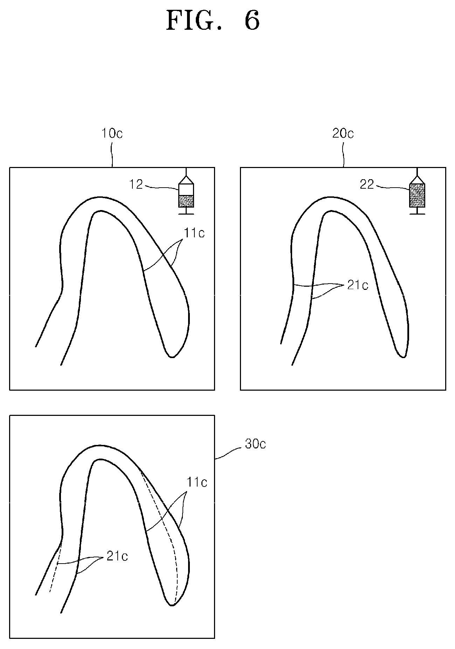

FIG. 6 illustrates first to third images 10c to 30c displayed on the display apparatus 120 of FIG. 1, according to another embodiment of the present invention.

Referring to FIG. 6, the display apparatus 120 (FIG. 1) may display the first image 10c and the second image 20c along with the third image 30c. The user may intuitively perceive a difference in thickness of the heart wall between when the subject is subjected to a first stress intensity level and when the subject is subjected to a second stress intensity level on the first to third images 10c to 30c.

The first image 10c and the second image 20c may further include a first marker 12 and a second marker 22, respectively, representing stress intensity levels the subject is subjected to, in addition to the first contour 11c and the second contour 21 c. The first marker 12 and the second marker 22 may display the stress intensity level the subject is subjected to as at least one of an image and text. As illustrated in FIG. 6, the first marker 12 may represent the first stress intensity level by displaying an image of administration of a low drug dosage, and the second marker 22 may represent the second stress intensity level higher than the first intensity level, by displaying an image of administration of a large drug dosage.

The user may readily perceive the first image 10c corresponding to the first stress intensity level and the second image 20c corresponding to the second stress intensity level from the first marker 12 and the second marker 22.

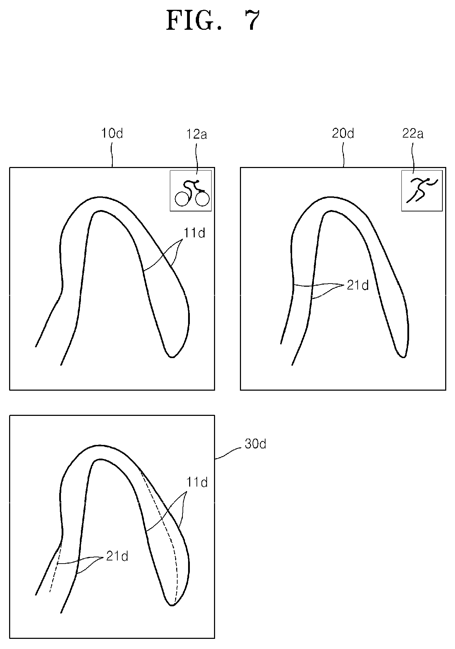

FIG. 7 illustrates first to third images 10d to 30d displayed on the display apparatus 120 of FIG. 1.

Referring to FIG. 7, the first image 10d and the second image 20d may include a first marker 12a and a second marker 22a, respectively, representing stress intensity levels then subject is subjected to. The first marker 12a may represent the first stress intensity level by displaying an image of riding a bike, and the second marker 22a may represent the second stress intensity level, higher than the first intensity level, by displaying an image of walking or running.

The first markers 12 and 12a and the second markers 22 and 22a in FIGS. 6 and 7 are only for illustrative purposes, not for limiting the embodiments thereof. The first markers 12 and 12a and the second markers 22 and 22a may display the stress intensity level the subject is subjected to in a variety of manners.

Referring back to FIGS. 1 and 3, the image processing apparatus 110 may acquire first data of the heart 210 of the subject 200 based on the first image data of the subject 200 acquired when the subject 200 is subjected to a first stress intensity level, and may acquire second data of the heart 210 based on the second image data acquired when the subject 200 is subjected to a second stress intensity level higher than the first stress intensity level. The image processing apparatus 110 may acquire a fourth image representing a difference between the first data and the second data. The display apparatus 120 may display the fourth image along with the first to third images 10 to 30.

The first data and the second data may be used to evaluate the functions of the heart 210. For example, the first data and the second data may be heart strain, a strain rate as strain with respect to time obtained by dividing strain by time, a heart wall thickness, a cardiac muscle change rate, or a heart volume, etc.

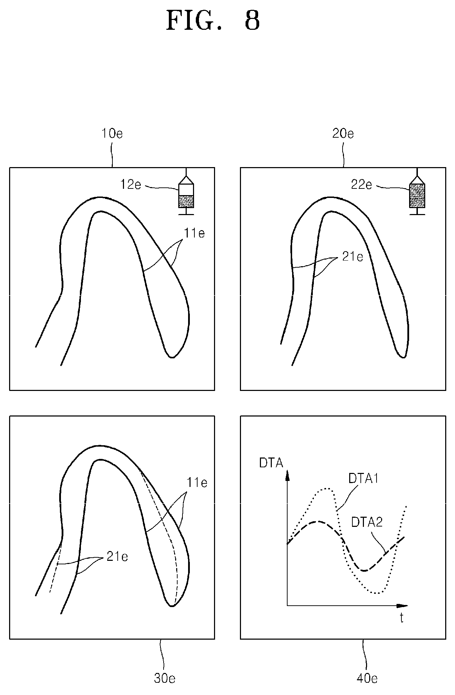

FIG. 8 illustrates first to fourth images 10e to 40e displayed on the display apparatus 120 of FIG. 1, according to another embodiment of the present invention.

Referring to FIG. 8, the display apparatus 120 (see FIG. 1) may display the fourth image 40e along with the first to third images 10e to 30e. The above-descriptions of the first to third images may be referred to herein to avoid redundancy.

The fourth image 40e may include a graph of first data DTA1 and second data DTA2 with respect to time t, wherein the time t may be a frame. The user may more easily analyze a difference between the first image 10e and the second image 20e from the fourth image 40e.

Although in FIG. 8 the fourth image 40e illustrates one piece of first data DTA1 and one piece of second data DTA2, the fourth image 40e may display a plurality of pieces of first data DTA1 and a plurality of pieces of second data DTA2. The first image 10e and the second image 20e may be represented with a plurality of pieces of first data DTA1 and a plurality of pieces of second data DTA2 that are assigned to a plurality of segments of the heart, respectively, as data for evaluating functions of the heart.

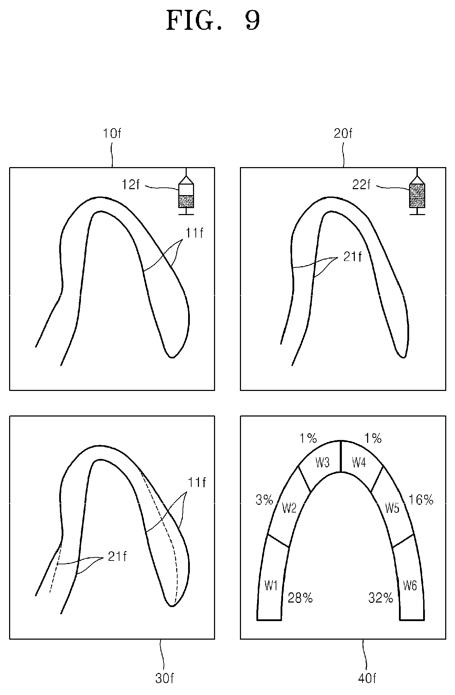

FIG. 9 illustrates first to fourth images 10f to 40f displayed on the display apparatus 120 of FIG. 1, according to another embodiment of the present invention.

Referring to FIG. 9, the fourth image 40f may represent a plurality of segments W1-W6 of the heart, each with a quantitative difference between the first data and the second data. In FIG. 9, the first data and the second data may be heart wall thicknesses. For example, the second segment W2 has a difference of about 3% between a heart wall thickness corresponding to the stress at the first intensity level and a heart wall thickness corresponding to the stress at the second intensity level higher than the first intensity level.

Further to the embodiments of FIGS. 8 and 9, a fourth image representing a difference between the first data corresponding to the first intensity level of stress and the second data corresponding to the second intensity level of stress may be displayed in any of a variety of manners depending on the types of the first and second data to evaluate functions of the heart.

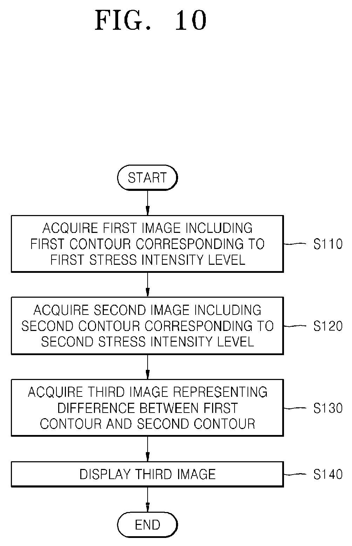

FIG. 10 is a flowchart of an operation method of a diagnosis imaging apparatus, according to an embodiment of the present invention.

Referring to FIG. 10, the diagnosis imaging apparatus may acquire a first image including a first contour of the heart wall based on first image data of the heart of the subject acquired when the subject is subjected to stress at a first intensity level (Operation S110). The diagnosis imaging apparatus may acquire a second image including a second contour of the heart wall based on second image data of the heart of the subject acquired when the subject is subjected to at a second stress intensity level higher than the first stress intensity level (Operation S120). The diagnosis imaging apparatus may acquire a third image representing a difference between the first contour and the second contour based on the first image and the second image (Operation S130). The diagnosis imaging apparatus may display the third image (Operation S140).

The operation method of FIG. 10 may be performed by the diagnosis imaging apparatus 100 of FIG. 1. The relevant descriptions described above with reference to FIGS. 1 to 9 may be referred to as a description of each operation of the method.

According to the embodiments described above, diagnosis imaging apparatuses and operating methods thereof are efficient.

The embodiments of the method described above may be written as computer programs and can be implemented in general-use digital computers that execute the programs using a computer-readable recording medium. Data used in the above-described embodiments can be recorded on a medium in various means. Examples of the computer-readable recording medium include magnetic storage media (e.g., ROM, RAM, USB, floppy disks, hard disks, etc.), optical recording media (e.g., CD-ROMs, or DVDs), and peripheral component interfaces (PCI) (e.g., PCI-express, or Wifi).

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A diagnosis imaging apparatus (100) comprising:
an image processing apparatus (110) for acquiring a first image (10) including a first contour (11) of a heart wall based on first image data of a heart (210) of a subject (200) acquired when the subject (200) is subjected to a first stress intensity level, acquiring a second image (20) including a second contour (21) of the heart wall based on second image data of the heart (210) of the subject (200) acquired when the subject (200) is subjected to a second stress intensity level, and acquiring a third image (30a) representing overlapping of the first contour (11) included in the first image (10) with the second contour (21) included in the second image (20); and
a display apparatus (120) for displaying the third image (30a),
**characterised in that** the second stress intensity level is different from the first stress intensity level.

2. The diagnosis imaging apparatus (100) of claim 1, wherein the display apparatus (120) displays the first image (10) and the second image (20) along with the third image (30a).

3. The diagnosis imaging apparatus (100) of claim 1, wherein the first image (10) and the second image (20) each further comprise a marker indicating a stress intensity level the subject (200) is subjected to.

4. The diagnosis imaging apparatus (100) of claim 1, wherein the image processing apparatus (110) acquires first data of the heart (210) based on the first image data, second data of the heart (210) based on the second image data, and a fourth image representing a difference between the first data and the second data; and the display apparatus (120) displays the fourth image along with the first to third images (30a).

5. The diagnosis imaging apparatus (100) of claim 4, wherein the first data and the second data each include at least one of heart strain, a strain rate, a heart wall thickness, a cardiac muscle change rate, and a heart volume.

6. The diagnosis imaging apparatus (100) of claim 5, wherein the fourth image includes a graph of the first data and the second data with respect to time.

7. The diagnosis imaging apparatus (100) of claim 5, wherein the first data and the second data each has a plurality of pieces of data corresponding to a plurality of segments of the hearts (210).

8. The diagnosis imaging apparatus (100) of claim 7, wherein the fourth image (10) represents the plurality of segments of the heart (210) each with a quantitative difference between the first data and the second data.

9. The diagnosis imaging apparatus (100) of claim 1, wherein the stress at the first intensity level and the stress at the second intensity level are applied by administering different drug dosages to the subject (200), or by the subject (200) exercising at different intensity levels.

10. An operation method of a diagnosis imaging apparatus (100), the method comprising:
acquiring a first image (10) including a first contour (11) of a heart wall based on first image data of a heart (210) of a subject (200) acquired when the subject (200) is subjected to a first stress intensity level;
acquiring a second image (20) including a second contour (21) of the heart wall based on second image data of the heart (210) of the subject (200) acquired when the subject (200) is subjected to a second stress intensity level;
the first and second stress intensity levels not being applied by administering drug dosages to the subject (200);
acquiring a third image (30a) representing overlapping of the first contour (11) included in the first image (10) with the second contour (21) included in the second image (20); and
displaying the third image (30a), **characterised in that** the second stress intensity level is different from the first stress intensity level.

11. The operation method of claim 10, further comprising:
acquiring first data of the heart (210) based on the first image data;
acquiring second data of the heart (210) based on the second image data;
acquiring a fourth image representing a difference between the first data and the second data; and
displaying the fourth image along with the third image (30a).

12. The operation method of claim 11, wherein the first data and the second data each include at least one of heart strain, a strain rate, a heart wall thickness, a cardiac muscle change rate, and a heart volume.

13. A computer-readable recording medium having embodied thereon a program for executing the operation method of a diagnosis imaging apparatus (100) according to any one of claims 10 to 12.

## Patentansprüche

1. Diagnosebildvorrichtung (100), welche Folgendes aufweist:
eine Bildverarbeitungseinrichtung (110) zum Erlangen eines ersten Bilds (10), welches eine erste Kontur (11) einer Herzwand basierend auf ersten Bilddaten eines Herzens (210) eines Subjekts (200) beinhaltet, die erlangt werden, wenn das Subjekt (200) einem ersten Belastungsintensitätsniveau unterzogen wird, Erlangen eines zweiten Bilds (20), welches eine zweite Kontur (21) der Herzwand basierend auf zweiten Bilddaten des Herzens (210) des Subjekts (200) beinhaltet, die erlangt werden, wenn das Subjekt (200) einem zweiten Belastungsintensitätsniveau unterzogen wird, und Erlangen eines dritten Bilds (30a), das ein Überlappen der in dem ersten Bild (10) enthaltenen ersten Kontur (11) mit der in dem zweiten Bild (20) enthaltenen zweiten Kontur (21) repräsentiert; und
eine Anzeigeeinrichtung (120) zum Anzeigen des dritten Bilds (30a),
**dadurch gekennzeichnet , dass**
das zweite Belastungsintensitätsniveau unterschiedlich von dem ersten Belastungsintensitätsniveau ist.

2. Diagnosebildvorrichtung (100) nach Anspruch 1, wobei die Anzeigeeinrichtung (120) das erste Bild (10) und das zweite Bild (20) zusammen mit dem dritten Bild (30a) anzeigt.

3. Diagnosebildvorrichtung (100) nach Anspruch 1, wobei das erste Bild (10) und das zweite Bild (20) jeweils des Weiteren eine Markierung aufweisen, die ein Belastungsintensitätsniveau anzeigt, welchem das Subjekt (200) ausgesetzt ist.

4. Diagnosebildvorrichtung (100) nach Anspruch 1, wobei die Bildverarbeitungseinrichtung (110) erste Daten des Herzens (210) basierend auf den ersten Bilddaten, zweite Daten des Herzens (210) basierend auf den zweiten Bilddaten und ein viertes Bild erlangt, das einen Unterschied zwischen den ersten Daten und den zweiten Daten repräsentiert;
und die Anzeigeeinrichtung (120) das vierte Bild zusammen mit den ersten bis dritten Bildern (30a) anzeigt.

5. Diagnosebildvorrichtung (100) nach Anspruch 4, wobei die ersten Daten und die zweiten Daten jeweils wenigstens eines einer Herzbelastung, einer Betastungsrate, einer Herzwanddicke, einer Herzmuskelveränderungsrate und eines Herzvolumens beinhalten.

6. Diagnosebildvorrichtung (100) nach Anspruch 5, wobei das vierte Bild einen Graph der ersten Daten und der zweiten Daten in Bezug auf die Zeit aufweist.

7. Diagnosebildvorrichtung (100) nach Anspruch 5, wobei die ersten Daten und die zweiten Daten jeweils eine Vielzahl von Teilen von Daten aufweisen, die einer Vielzahl von Segmenten des Herzens (210) entsprechen.

8. Diagnosebildvorrichtung (100) nach Anspruch 7, wobei das vierte Bild (10) die Vielzahl von Segmenten des Herzens (210) jeweils mit einer quantitativen Differenz zwischen den ersten Daten und den zweiten Daten repräsentiert.

9. Diagnosebildvorrichtung (100) nach Anspruch 1, wobei die Belastung auf dem ersten Intensitätsniveau und die Belastung auf dem zweiten Intensitätsniveau durch Verabreichen unterschiedlicher Dosierungen von Medikamenten an das Subjekt (200) oder dadurch, dass das Subjekt (200) auf unterschiedlichen Intensitätsniveaus trainiert, aufgebracht wird.

10. Betriebsverfahren einer Diagnosebildvorrichtung (100), wobei das Verfahren Folgendes aufweist:
Erlangen eines ersten Bilds (10), welches eine erste Kontur (11) einer Herzwand basierend auf ersten Bilddaten eines Herzens (210) eines Subjekts (200) beinhaltet, die erlangt werden, wenn das Subjekt (200) einem ersten Belastungsintensitätsniveau unterzogen wird;
Erlangen eines zweiten Bilds (20), welches eine zweite Kontur (21) der Herzwand basierend auf zweiten Bilddaten des Herzens (210) des Subjekts (200) beinhaltet, die erlangt werden, wenn das Subjekt (200) einem zweiten Belastungsintensitätsniveau unterzogen wird,
wobei die ersten und zweiten Belastungsintensitätsniveaus nicht durch Verabreichen von Dosierungen von Medikamenten auf das Subjekt (200) aufgebracht werden;
Erlangen eines dritten Bilds (30a), das ein Überlappen der in dem ersten Bild (10) enthaltenen ersten Kontur (11) mit der in dem zweiten Bild (20) enthaltenen zweiten Kontur (21) repräsentiert; und
Anzeigen des dritten Bilds (30a),
**dadurch gekennzeichnet, dass**
das zweite Belastungsintensitätsniveau unterschiedlich von dem ersten Belastungsintensitätsniveau ist.

11. Betriebsverfahren nach Anspruch 10, welches des Weiteren Folgendes aufweist:
Erlangen erster Daten des Herzens (210) basierend auf den ersten Bilddaten;
Erlangen zweiter Daten des Herzens (210) basierend auf den zweiten Bilddaten;
Erlangen eines vierten Bilds, das einen Unterschied zwischen den ersten Daten und den zweiten Daten repräsentiert; und
Anzeigen des vierten Bilds zusammen mit dem dritten Bild (30a).

12. Betriebsverfahren nach Anspruch 11, wobei die ersten Daten und die zweiten Daten jeweils wenigstens eines einer Herzbelastung, einer Belastungsrate, einer Herzwanddicke, einer Herzmuskelveränderungsrate und eines Herzvolumens beinhalten.

13. Computerlesbares Aufzeichnungsmedium, auf dem ein Programm zur Durchführung des Betriebsverfahrens einer Diagnosebildvorrichtung (100) nach einem der Ansprüche 10 bis 12 enthalten ist.

## Revendications

1. Appareil (100) d'imagerie pour diagnostic comportant :
un appareil de traitement d'image (110) pour acquérir une première image (10) comprenant un premier contour (11) d'une paroi cardiaque basée sur des premières données d'image d'un coeur (210) d'un patient (200) acquises lorsque le patient (200) est soumis à un premier degré d'intensité d'effort, acquérir une deuxième image (20) comprenant un deuxième contour (21) de la paroi cardiaque basée sur des deuxièmes données d'image du coeur (210) du patient (200) acquises lorsque le patient (200) est soumis à un deuxième degré d'intensité d'effort, et acquérir une troisième image (30a) représentant la superposition du premier contour (11) inclus dans la première image (10) avec le deuxième contour (21) inclus dans la deuxième image(20) ; et
un appareil d'affichage (120) pour afficher la troisième image (30a), **caractérisé en ce que** le deuxième degré d'intensité d'effort est différent du premier degré d'intensité d'effort.

2. Appareil (100) d'imagerie pour diagnostic selon la revendication 1, dans lequel l'appareil d'affichage (120) affiche la première image (10) et la deuxième image (20) simultanément avec la troisième image (30a).

3. Appareil (100) d'imagerie pour diagnostic selon la revendication 1, dans lequel la première image (10) et la deuxième image (20) comprennent chacune en outre un marqueur indiquant un degré d'intensité d'effort auquel le patient (200) est soumis.

4. Appareil (100) d'imagerie pour diagnostic selon la revendication 1, dans lequel l'appareil de traitement d'image (110) acquiert des premières données relatives au coeur (210) basées sur les premières données d'image, des deuxièmes données du coeur (210) basées sur les deuxièmes données d'image, et une quatrième image représentant la différence entre les premières données et les deuxièmes données ; et l'appareil d'affichage (120) affichant la quatrième image simultanément avec la première à la troisième image (30a).

5. Appareil (100) d'imagerie pour diagnostic selon la revendication 4, dans lequel les premières et les deuxièmes données comportent chacune au moins une des données comprenant un effort cardiaque, un rythme cardiaque, une épaisseur des parois du coeur, un changement du rythme des battements du muscle cardiaque, et un volume du coeur.

6. Appareil (100) d'imagerie pour diagnostic selon la revendication 5, dans lequel la quatrième image comporte un graphique représentant les premières données et les deuxièmes données en fonction du temps.

7. Appareil (100) d'imagerie pour diagnostic selon la revendication 5, dans lequel les premières données et les deuxièmes données comportent chacune une pluralité de pièces de données correspondant à une pluralité de segments du coeur (210).

8. Appareil (100) d'imagerie pour diagnostic selon la revendication 7, dans lequel la quatrième image (10) représente la pluralité de segments du coeur (210), chacun avec une différence quantitative entre les premières données et les deuxièmes données.

9. Appareil (100) d'imagerie pour diagnostic selon la revendication 1, dans lequel le premier degré d'intensité d'effort, et le deuxième degré d'intensité d'effort sont appliqués en administrant différentes doses de médicaments au patient (200), ou en faisant effectuer des efforts de différentes intensités au patient (200).

10. Méthode opératoire d'un appareil (100) d'imagerie pour diagnostic, cette méthode comprenant :
l'acquisition d'une première image (10) comprenant un premier contour (11) d'une paroi cardiaque basée sur des premières données d'image d'un coeur (210) d'un patient (200) acquises lorsque le patient (200) est soumis à un premier degré d'intensité d'effort,
l'acquisition d'une deuxième image (20) comprenant un deuxième contour (21) de la paroi cardiaque basée sur des deuxièmes données d'image du coeur (210) du patient (200) acquises lorsque le patient (200) est soumis à un deuxième degré d'intensité d'effort,
le premier et le deuxième degrés d'intensité d'effort, n'étant pas appliqués par l'administration de doses différentes de médicaments au patient (200) ;
l'acquisition d'une troisième image (30a) représentant la superposition du premier contour (11) incluant la première image (10) avec le deuxième contour (21) incluant le deuxième contour (20) ; et
l'affichage de la troisième image (30a), **caractérisé en ce que** le premier degré d'intensité d'effort est différent du deuxième degré d'intensité d'effort.

11. Méthode opératoire selon la revendication 10, comportant en outre:
l'acquisition des premières données du coeur (210) basées sur les premières données d'image ;
l'acquisition des deuxièmes données du coeur (210) basées sur les deuxièmes données d'image ;
l'acquisition de la quatrième image représentant la différence entre les premières données et les deuxièmes données ; et
l'affichage de la quatrième image en même temps que la troisième image (30a).

12. Méthode opératoire selon la revendication 11, dans lequel les premières et les deuxièmes données comportent chacune au moins une des données comprenant un effort cardiaque, un rythme cardiaque, une épaisseur des parois du coeur, un changement du rythme des battements du muscle cardiaque, et un volume du coeur.

13. Support de données pouvant être lues par un ordinateur, sur lequel est enregistré un programme pour exécuter la méthode opérationnelle sur un appareil (100) d'imagerie pour diagnostic selon l'une quelconque des revendications 10 à 12.
